(19) Europäisches Patentamt
European Patent Office
Office européen des brevets

(11) Veröffentlichungsnummer: 0 230 863
A2

(12) # EUROPÄISCHE PATENTANMELDUNG

(21) Anmeldenummer: 86810621.2

(22) Anmeldetag: 31.12.86

(51) Int.Cl.³: C 07 D 273/00
A 01 N 43/88

(30) Priorität: 09.01.86 CH 37/86
28.11.86 CH 4768/86

(43) Veröffentlichungstag der Anmeldung:
05.08.87 Patentblatt 87/32

(84) Benannte Vertragsstaaten:
AT BE CH DE ES FR GB IT LI NL

(71) Anmelder: CIBA-GEIGY AG
Klybeckstrasse 141
CH-4002 Basel(CH)

(72) Erfinder: Farooq, Saleem, Dr.
Kirchackerstrasse 27
CH-4422 Arisdorf(CH)

(72) Erfinder: Kühne, Manfred, Dr.
Alemannenweg 11
CH-4148 Pfeffingen(CH)

(54) **Oxadiazinone.**

(57) Neue substituierte 1,3,5-Oxadiazin-2-one der Formel

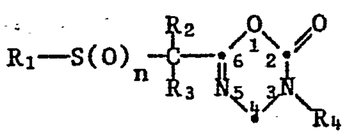

(I),

worin

$R_1$ und $R_4$ unabhängig voneinander $C_1$-$C_8$-Alkyl, $C_3$-$C_6$-Cycloalkyl, $C_3$-$C_4$-Alkenyl, $C_3$-$C_4$-Alkinyl, Phenyl oder Benzyl;

$R_2$ und $R_3$ unabhängig voneinander Wasserstoff, $C_1$-$C_4$-Alkyl oder $R_2$ und $R_3$ zusammen mit dem Kohlenstoffatom, an das sie gebunden sind, $C_3$-$C_6$-Cycloalkyl bedeuten; und

n = 0, 1 oder 2 ist; Verfahren zur Herstellung dieser Verbindungen sowie diese enthaltende Mittel zur Verwendung in der Schädlingsbekämpfung, insbesondere zur Bekämpfung von Pflanzen und Tiere befallenden Insekten und Vertretern der Ordnung Akarina. Die neuen Verbindungen weisen insbesondere hohe Wirksamkeit gegen pflanzenschädigende saugende und fressende Insekten auf.

EP 0 230 863 A2

Croydon Printing Company Ltd.

CIBA-GEIGY AG
Basel (Schweiz)

5-15702/1+2

# Oxadiazinone

Die vorliegende Erfindung betrifft neuartige substituierte 6-Sulfenylmethyl-, 6-Sulfinylmethyl- und 6-Sulfonylmethyl-1,3,5-oxadiazin-2-one Verfahren zu ihrer Herstellung und ihre Verwendung in der Schädlingsbekämpfung.

Diese erfindungsgemässen Verbindungen haben die Formel I

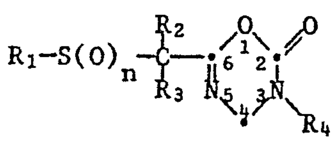

(I),

worin

$R_1$ und $R_4$ unabhängig voneinander $C_1$-$C_8$-Alkyl, $C_3$-$C_6$-Cycloalkyl, $C_3$-$C_4$-Alkenyl, $C_3$-$C_4$-Alkinyl, Phenyl oder Benzyl;

$R_2$ und $R_3$ unabhängig voneinander Wasserstoff, $C_1$-$C_4$-Alkyl oder $R_2$ und $R_3$ zusammen mit dem Kohlenstoffatom, an das sie gebunden sind, $C_3$-$C_6$-Cycloalkyl bedeuten; und

n = 0, 1 oder 2 ist.

Unter dem Begriff Alkyl sind im Rahmen der vorliegenden Erfindung geradkettige und verzweigte Alkylreste und je nach Zahl der angegebenen Kohlenstoffatome beispielsweise folgende Gruppen zu verstehen: Methyl, Aethyl, Propyl, Butyl, Pentyl, Hexyl usw., sowie ihre Isomeren, wie z.B. Isopropyl, Isobutyl, tert.-Butyl, Isopentyl usw.

Wegen ihrer Wirkung als Schädlingsbekämpfungsmittel bevorzugt sind Verbindungen der Formel I, die dadurch gekennzeichnet sind, dass

$R_1$ $C_1$-$C_4$-Alkyl oder Phenyl;

$R_2$ und $R_3$ Methyl;

$R_4$ $C_1$-$C_4$-Alkyl bedeuten; und

n = 0, 1 oder 2 ist.

Wertvoll sind aufgrund ihrer biologischen Wirksamkeit ferner solche Verbindungen der Formel I, worin

$R_1$ Methyl oder Aethyl;

$R_2$ und $R_3$ Methyl;

$R_4$ $C_1$-$C_3$-Alkyl bedeuten; und

n = 0 oder 1 ist.

Die Verbindungen der Formel I können hergestellt werden, indem man ein entsprechend substituiertes N-Aminomethylacetamid der Formel II

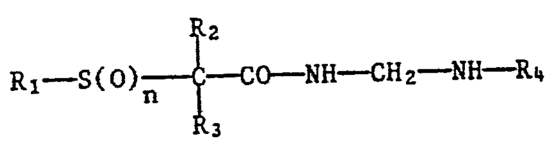

(II)

durch Umsetzung mit Phosgen cyclisiert, wobei die Reste $R_1$ bis $R_4$ und n die vorstehend angegebenen Bedeutungen haben. Dieses Verfahren kann auch in dem Sinne modifiziert werden, dass man eine Verbindung der Formel II durch Umsetzung eines in 1,3,5-Stellung entsprechend substituierten 2,4,6-Hexahydrotriazins der Formel III

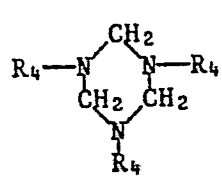

(III)

mit einem Acetamid der Formel IV

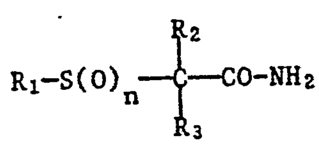

(IV)

in situ herstellt und dann direkt mit Phosgen umsetzt, wobei in den Formeln III und IV die Reste $R_1$ bis $R_4$ und n die vorstehend angegebenen Bedeutungen haben.

Die oben erwähnte Cyclisierung wird durchgeführt, indem man das Aminomethylacetamid der Formel II bei Temperaturen zwischen -50°C und +30°C mit Phosgen, vorzugsweise in Gegenwart einer Base und in gegenüber den Reaktionsteilnehmern inerten Lösungs- und/oder Verdünnungsmitteln, umsetzt und anschliessend, vorzugsweise in Gegenwart einer Base, bei Temperaturen zwischen -15° und +120°, gewünschtenfalls auch unter Druck, den Ringschluss vornimmt.

Als Lösungs- oder Verdünnungsmittel kommen dabei aliphatische oder aromatische Kohlenwasserstoffe wie Benzol, Toluol, Xylole, Hexan; Halogenkohlenwasserstoffe wie Chloroform, Methylenchlorid; Ketone, wie Aceton, Methyläthylketon; Nitrile wie Acetonitril; Dimethylformamid oder Dimethylsulfon; insbesondere jedoch Aether und ätherartige Verbindungen, wie Dialkyläther, Dioxan, Tetrahydrofuran, 1,2-Dimethoxyäthan sowie zweiphasige Gemische, z.B. Wasser/Benzol, in Betracht. Als Basen sind insbesondere tertiäre Amine, z.B. Trialkylamine, Pyridin oder Pyridinbasen, aber auch NaH oder im Falle wasserhaltiger Gemische Alkali- oder Erdalkalihydroxyde oder -carbonate zu nennen.

Wie vorstehend angegeben lassen sich die Verbindungen der Formel I vorteilhafterweise auch im Eintopfverfahren (in situ) direkt aus Acetamiden der Formel IV herstellen. Diese Verfahrensvariante führt man in der Weise durch, dass man das Acetamid der Formel IV in Gegenwart einer wasserfreien Säure, vorzugsweise einer Halogenwasserstoffsäure, z.B. HCl, mit einem substituierten Triazin der Formel III in einem inerten organischen Lösungsmittel umsetzt und anschliessend - ohne das Zwischenprodukt der Formel II zu isolieren - mit Phosgen, vorzugsweise in Gegenwart einer Base, die Cyclisierung durchführt. Diese spezielle Ausführungsform des Verfahrens zur Herstellung der Verbindungen der Formel I bietet den Vorteil, dass auch solche Verbindungen der Formel II, die säureempfindlich sind, ohne Schwierigkeiten cyclisiert werden können.

Die Ausgangsstoffe der vorstehenden Formel II, III und IV sind bekannt und lassen sich analog bekannten Verfahren in üblicher Weise herstellen (vgl. DE-OS 2 459 413 und 2 624 341; J. Agr. Food Chem. 18(3), S. 454-458, 1970).

Erhält man bei dem oben beschriebenen Herstellungsverfahren als Verbindungen der Formel I 6-Sulfenylmethyl- oder 6-Sulfinylmethylderivate, d.h. Verbindungen der Formel I, worin n = 0 oder 1 ist, so kann man diese erhaltenen Verbindungen in an sich bekannter Weise zu den entsprechenden 6-Sulfonylmethylderivaten, d.h. Verbindungen der Formel I, worin n = 2 ist, oxydieren. Als Oxydationsmittel kommen für diesen Zweck bevorzugt Peroxyverbindungen, wie $H_2O_2$ und Persäuren, z.B. p-Chlorperbenzoesäure oder Peressigsäure, in Betracht. In analoger Weise kann man Verbindungen der Formel I, worin n = 0 ist, zu Verbindungen der Formel I, worin n = 1 ist, oxydieren.

Es ist bereits aus der DE-OS 2 459 413 bekannt, 6-Phenyl-1,3,5-oxa-diazin-2-one, die in 3-Stellung gegebenenfalls durch Alkyl, Alkoxyalkyl, Alkenyl oder Cycloalkyl substituiert sind, als Herbizide und Coccidiostatika zu verwenden. Aehnliche Verbindungen werden in der CH-PS 630 245 als Beifuttermittel vorgeschlagen. Ferner werden in 6-Stellung durch einen heterozyklischen Rest substituierte 1,3,5-Oxadiazin-2-one und ihre Verwendung zur Bekämpfung von Coccidien in der DE-OS 2 624 341 beschrieben.

Bei den Verbindungen der Formel I gemäss vorliegender Erfindung handelt es sich demgegenüber um neuartige in 3,6-Stellung disubstituierte 1,3,5-Oxadiazin-2-one, deren kennzeichnendes Strukturmerkmal das Vorliegen einer substituierten Sulfenylmethyl-,

Sulfinylmethyl- oder Sulfonylmethylgruppe als Substituent in 6-Stellung darstellt. Es wurde überraschenderweise gefunden, dass die erfindungsgemässen Verbindungen der Formel I bei guter Pflanzenverträglichkeit und sehr geringer Warmblütertoxizität eine ausgeprägte Wirksamkeit als Schädlingsbekämpfungsmittel, insbesondere als Insektizide, besitzen. Die Verbindungen eignen sich vor allem zur Bekämpfung von Pflanzen und Tiere befallenden Schädlingen.

Insbesondere eignen sich die Verbindungen der Formel I zur Bekämpfung von Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera sowie von Vertretern der Ordnung Akarina.

Die gute pestizide Wirkung der erfindungsgemässen Verbindungen der Formel I entspricht einer Abtötungsrate (Mortalität) von mindestens 50-60 % der erwähnten Schädlinge.

Neben ihrer sehr günstigen Wirkung gegenüber Fliegen, wie z.B. Musca domestica, und Mücken eignen sich Verbindungen der Formel I vor allem zur Bekämpfung von pflanzenschädigenden saugenden Insekten und Frassinsekten, in Zier- und Nutzpflanzungen. Die Verbindungen der Formel I sind wirksam gegen larvale Insektenstadien und Nymphen, insbesondere von fressenden Schadinsekten. Hervorzuheben ist speziell die gute systemische und Kontakt-Wirkung der Verbindungen der Formel I, z.B. gegen Obst-, Reis- und Gemüseschädlinge. Werden Verbindungen der Formel I von adulten Insekten mit dem Futter aufgenommen, so ist in vielen Fällen, insbesondere bei Coleopteren, wie z.B. Anthonomus grandis, eine verminderte Ei-Ablage und/oder reduzierte Schlupfrate festzustellen. In diesem Zusammenhang ist hervorzuheben, dass die Verbindungen der Formel I sich sowohl durch eine stark ausgeprägte systemische als auch Kontakt-Wirkung gegen saugende Insekten, vor allem gegen Insekten der Familie Aphididae (wie z.B. Aphis fabae, Aphis craccivora und Myzus persicae), welche sich mit bekannten Mitteln nur schwierig bekämpfen lassen, auszeichnen. Auch können die Verbindungen der Formel I mit Erfolg gegen pflanzenschädigende Zikaden, speziell in Reis-Kulturen, wie z.B. Nilaparvata lugens, Laodelphax striatellus und Nephotettix, eingesetzt werden.

Die Verbindungen der Formel I eignen sich weiterhin zur Bekämpfung von Ektoparasiten, z.B. Lucilia sericata, sowie von Zecken an Haus- und Nutztieren, z.B. durch Tier-, Stall- und Weidebehandlung.

Es wurde weiterhin gefunden, dass Kombinationen von Verbindungen der Formel I mit anderen Insektiziden und/oder Akariziden (z.B. org. Phosphorverbindungen; Imidoäther; Amidine; Amine; Hydrazine; Triazine; Harnstoffe; pyrethrinartige Verbindungen; Diphenylmethanderivate und Karbamate) in einem Verhältnis von 5:1 bis 1:5, vorzugsweise 1:1, eine potenzierende bzw. synergierende Wirkung gegen verschiedenartige Schädlinge an Tieren und Pflanzen, insbesondere gegen pflanzenschädigende Insekten, entfalten, welche die additive Wirkung dieser kombinierten Wirkstoffe überraschenderweise stark übertrifft. Dieser potenzierende Effekt bzw. Synergismus der kombinierten Wirkstoffe tritt bei allen Bekämpfungsverfahren auf. So können Verfahrensweisen angewendet werden, wobei die Wirkstoffe vorgemischt aus einem Behälter oder zeitlich kurz nacheinander oder simultan aus verschiedenen Behältern in den Lebensraum der Schädlinge, bzw. auf diese selbst, appliziert werden.

Einen potenzierenden bzw. synergierenden Effekt erzeugen Verbindungen der Formel I insbesondere in Mischungen mit z.B. folgenden, bekannten Insektiziden oder Akariziden:

1. Org. Phosphorester:

1.1 Alkyl-(Acyl-)Phosphate:

Acephat; Methamidophos; Ethoprophos; Disulfoton; TEPP; Naled; Carbophenothion; Trichlorphon; Ethion; Sulfotep; Mipafox; Vamidothion; Phenkapton; Terbufos; Chlormephos; Phoxim; Phorat und Fospargyl.

1.2 Carbonylalkylphosphate:

Phenthoat; Dimethoat; Malathion; Morphothion und Formothion.

1.3 Vinylphosphate.

Tetrachlorvinphos; Propetamphos; Methacrifos; Chlorfenvinphos; Tetrachlorvinphos-äthyl; Thiophosdrin®; Mevinphos; Crotoxyphos; Phosphamidon; Dicrotophos; Monocrotophos; Dichlorvos; Fosfinon®; Akton®; Bomyl®, Bromfenvinphos und Heptenophos.

1.4 Aromatische Phosphate:

Profenofos; Trifenophos; Bromophos; Mercaptoprophos; Methylparathion; Parathion; Chlorthion; Fenitrothion; Fenchlorphos; Fenthion; Cyanophos; Dichlofenthion; Fenamiphos; Fonofos; Jodfenfos; Temephos; Prothiophos; Isofenphos; Fensulfothion und Lepthophos.

1.5 Heterocyclische Phosphate:

Isazophos; Triazophos; Chlorpyrifos; Phosmet; Azamethiphos; Phosalon; Azinphos-methyl; Azinphos-äthyl; Dialifos; Dioxathion; Diazinon; Methidathion; Isoxathion; Chlorpyrofos-methyl; Phosfolan; Fosthietan; Etrimfos; Pyridafenthion; Mephosfolan; Thionazin; Zolaprofos und Pyrimiphos-methyl.

2. Carbamate:

2.1 Aromatische Carbamate:

Dioxacarb; Methiocarb; Isoprocarb; Carbaryl; Xylylcarb; CPMC, Bufencarb; BPMC; Carbofuran; Propoxur, Ethiofencarb; Carbosulfan; Mydrol®; Bendiocarb; Aminocarb; Chloetocarb und Benzofuracarb.

2.2 Heterocyclische Carbamate:

Isolan®; Dimetilan; Primicarb; Hyquincarb; Methomyl; Aldicarb; Tirpat®; Demethyl-oxamyl; Thiodicarb; Nitrilacarb; Oxamyl; Thiofanox und Butocarboxim.

3. Diphenylmethanderivate:

DDT; Methoxychlor; Prolan; Bulan; Chlorbenzilat; Chlorpropylat und Brompropylat.

4. Amidine, Imidoäther, Amine, Hydrazine:
Chlordimeform; Benzomat und Amitraz.

5. Pyrethrinartige Verbindungen:
Resmethrin; Permethrin; Phenothrin; Cypermethrin; Decamethrin; Fenpropathrin; Fenfluthrin; Fenpyrithrin; Cyhalothrin; Flumethrin; Cyfluthrin; Fenvalerat; Fluvalinat und Flucytrin.

6. Harnstoff und Triazine.
Diflubenzuron; Flucarbenzuron und Cypromazin.

Die Verbindungen der Formel I oder deren Kombinationen oder synergistische Gemische mit anderen Wirkstoffen werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierung, d.h. die den Wirkstoff der Formel I, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder

Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von

natürlichen Fettsäuregemischen, die z.B. aus Kokosnuss- oder Talgöl gewonnen werden können. Ferner sind als Tenside auch die Fettsäuremethyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxidaddukte an Polypropylenglykol,

Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood, New Jersey, 1979;
Dr. Helmut Stache "Tensid Taschenbuch", Carl Hanser Verlag München/Wien 1981.

Die pestiziden Zubereitungen enthalten in der Regel 0,1 bis 99 %, insbesondere 0,1 bis 95 %, Wirkstoff der Formel I oder Kombinationen dieser Wirkstoffe mit andern Insektiziden oder Akariziden, 1 bis 99,9 % eines festen oder flüssigen Zusatzstoffes und 0 bis 25 %,

insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effeke enthalten.

Beispiel 1: Herstellung von 6-(1-Methyl-1-methylsulfenyläthyl)-2-isopropyl-3,4-dihydro-2H-1,3,5-oxadiazin-2-on:

In 300 ml Dimethoxyäthan werden bei -30°C 5 g gasförmiger Chlorwasserstoff eingeleitet und danach 6,4 g 1,3,5-Triisoproply-2,4,6-hexahydrotriazin hinzugefügt. Das Reaktionsgemisch wird bei -20°C gehalten, wobei 12 g α-Methylmercaptoisobuttersäureamid zugesetzt werden. Nach 2½ stündigem Rühren des Reaktionsgemisches bei Raumtemperatur werden, ebenfalls bei -20°C, 50 ml einer 20%igen Phosgenlösung in Toluol und anschliessend eine Lösung von 15 ml Pyridin in 30 ml Dimethoxyäthan tropfenweise zu dem Reaktionsgemisch hinzugefügt. Dann wird das Reaktionsgemisch während 1 Stunde bei Raumtemperatur gerührt. Anschliessend werden noch 30 ml Pyridin tropfenweise zugegeben und das Gemisch 4 Stunden bei 60°C weitergerührt. Zur Aufarbeitung wird das Reaktionsgemisch abgekühlt, filtriert und eingedampft. Der erhaltene Rückstand wird in Essigester aufgenommen, zweimal mit Wasser und zweimal mit einer gesättigten Kochsalzlösung ausgeschüttelt. Die abgetrennte organische Phase wird über Natriumsulfat getrocknet und eingedampft. Der Rückstand wird in Hexan aufgenommen, mit wenig Kieselgel versetzt, abfiltriert und eingedampft. Man erhält so die Titelverbindung der Formel

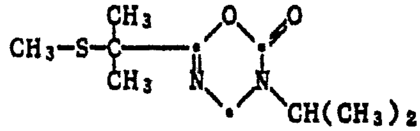

mit einem Brechungsindex $n_D^{20}$ = 1,5028 (Verbindung Nr. 1).

Beispiel 2: Herstellung von 6-(1-Methyl-1-methylsulfinyläthyl)-3-isopropyl-3,4-dihydro-2H-1,3,5-oxadiazin-2-on:

Es werden 11,5 g 6-(1-Methyl-1-methylmercaptoäthyl)-3-isopropyl-3,4-dihydro-2H-1,3,5-oxadiazin-2-on (Beispiel 1) in 100 ml Chloroform gelöst und auf -10°C abgekühlt. Zu dieser Lösung werden 9,5 g m-Chlorperbenzoesäure gelöst in 120 ml Chloroform zugetropft. Anschliessend wird das Reaktionsgemisch eine Stunde bei Raumtemperatur gerührt und dann eingeengt. Das zurückbleibende Rohprodukt wird aus Essigester:Hexan (1:1) umkristallisiert, und man erhält die Titelverbindung der Formel

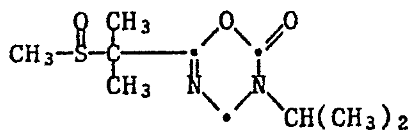

mit einem Schmelzpunkt von 120°-121°C (Verbindung Nr. 2).

Beispiel 3: Herstellung von 6-(1-Methyl-1-methylsulfonyläthyl)-3-methyl-3,4-dihydro-2H-1,3,5-oxadiazin-2-on:

Es werden 20,2 g 6-(1-Methyl-1-methylmercaptoäthyl)-3-methyl-3,4-dihydro-2H-1,3,5-oxadiazin-2-on in 100 ml Chloroform gelöst und auf 0°C abgekühlt. Dann werden zu der abgekühlten Lösung 16 ml einer 40%igen Peressigsäurelösung während etwa 15 Minuten zugetropft, wobei man die Reaktionstemperatur auf ca. 40°C ansteigen lässt und danach weitere 16 ml 40%ige Peressigsäurelösung hinzufügt. Das Reaktionsgemisch wird noch 10 Stunden bei Raumtemperatur gerührt und anschliessend auf ein Eis-Wasser-Gemisch gegossen. Die abgetrennte Chloroformphase wird zweimal mit Wasser gewaschen, über $Na_2SO_4$ getrocknet, abfiltriert und eingeengt. Das als Rückstand verbleibende Rohprodukt wird mit wenig Wasser gewaschen und über KOH bei 40°C getrocknet. Man erhält die erwünschte Titelverbindung der Formel

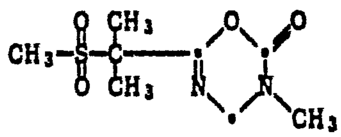

mit einem Schmelzpunkt von 134°-136°C (Verbindung Nr. 3).

Wie vorstehend beschrieben werden die folgenden Verbindungen der Formel I hergestellt:

| Verbindung Nr. | $R_1$ | $R_2$ | $R_3$ | $R_4$ | n | physikalische Daten |
|---|---|---|---|---|---|---|
| 4 | $CH_3-$ | $CH_3-$ | $CH_3-$ | $CH_3-$ | 0 | Smp. 86-87°C |
| 5 | $CH_3-$ | $CH_3-$ | $CH_3-$ | $CH_3-$ | 1 | Smp. 84-89°C |
| 6 | $n-C_8H_{17}-$ | $CH_3-$ | $CH_3-$ | $CH_3-$ | 0 | Smp. 33-34°C |
| 7 | $C_2H_5-$ | $CH_3-$ | $CH_3-$ | $CH_3-$ | 0 | Smp. 49-50°C |
| 8 | $C_2H_5-$ | $CH_3-$ | $CH_3-$ | $CH_3-$ | 1 | Smp. 66-68°C |
| 9 | $n-C_8H_{17}-$ | $CH_3-$ | $CH_3-$ | $CH_3-$ | 1 | klares Oel |
| 10 | $i-C_3H_7-$ | $CH_3-$ | $CH_3-$ | $CH_3-$ | 0 | Smp. 84-87°C |
| 11 | $i-C_3H_7-$ | $CH_3-$ | $CH_3-$ | $CH_3-$ | 1 | Smp. 76-78°C |
| 12 | $n-C_4H_9-$ | $CH_3-$ | $CH_3-$ | $CH_3-$ | 0 | helles Oel |
| 13 | 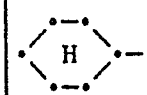 | $CH_3-$ | $CH_3-$ | $CH_3-$ | 0 | Smp. 68-70°C |
| 14 | $CH_2=CH-CH_2-$ | $CH_3-$ | $CH_3-$ | $CH_3-$ | 0 | Smp. 38-40°C |
| 15 | $CH_3-$ | $CH_3-$ | $CH_3-$ | $n-C_3H_7-$ | 0 | $n_D^{20}$ = 1,5051 |
| 16 | $CH_3-$ | $CH_3-$ | $CH_3-$ | $C_2H_5-$ | 0 | Smp. 38-40°C |
| 17 | $CH_3-$ | $CH_3-$ | $CH_3-$ | $n-C_4H_9-$ | 0 | $n_D^{20}$ = 1,5522 |
| 18 | $CH_3-$ | $CH_3-$ | $CH_3-$ | $s-C_4H_9-$ | 0 | $n_D^{20}$ = 1,4929 |
| 19 | $CH_3-$ | $CH_3-$ | $CH_3-$ | $i-C_4H_9-$ | 0 | $n_D^{20}$ = 1,4989 |
| 20 | $CH_3-$ | $CH_3-$ | $CH_3-$ | $t-C_4H_9-$ | 0 | $n_D^{20}$ =1,4994 |
| 21 | $CH_3-$ | $CH_3-$ | $CH_3-$ | $n-C_8H_{17}-$ | 0 | $n_D^{20}$ =1,4872 |
| 22 | $CH_3-$ | $CH_3-$ | $CH_3-$ | 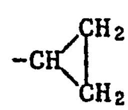 | 0 | Smp. 72-73°C |
| 23 | $C_2H_5-$ | $CH_3-$ | $CH_3-$ | $i-C_3H_7-$ | 0 | $n_D^{20}$ = 1,4982 |
| 24 | $i-C_3H_7-$ | $CH_3-$ | $CH_3-$ | $i-C_3H_7-$ | 0 | $n_D^{20}$ = 1,4942 |

In entsprechender Weise wie vorstehend angegeben sind auch die folgenden Verbindungen der Formel I herstellbar:

| Verbindung Nr. | $R_1$ | $R_2$ | $R_3$ | $R_4$ | n | physikalische Daten |
|---|---|---|---|---|---|---|
| 25 | $CH_3-$ | $CH_3-$ | $CH_3-$ | $i-C_3H_7-$ | 2 | |
| 26 | $CH_3-$ | $CH_3-$ | $CH_3-$ | $i-C_4H_9-$ | 1 | |
| 27 | $CH_3-$ | $CH_2-CH_2-$ | | $CH_3-$ | 0 | Smp. 61-63°C |
| 28 | $CH_3-$ | $CH_2-CH_2-$ | | $CH_3-$ | 1 | |
| 29 | $CH_3-$ | $CH_2-CH_2-$ | | $CH_3-$ | 2 | |
| 30 | $CH_3-$ | $CH_2-CH_2-$ | | $i-C_3H_7-$ | 0 | $n_D^{20}$ = 1,5151 |
| 31 | $CH_3-$ | $CH_2-CH_2-$ | | $i-C_3H_7-$ | 1 | Smp.: 117-119° |
| 32 | $CH_3-$ | $CH_2-CH_2-$ | | $i-C_3H_7-$ | 2 | |
| 33 | $CH_3-$ | $-CH_2-(CH_2)_3-CH_2-$ | | $C_2H_5-$ | 0 | |
| 34 | $CH_3-$ | $-CH_2-(CH_2)_3-CH_2-$ | | $C_2H_5-$ | 1 | |
| 35 | $CH_3-$ | $-CH_2-(CH_2)_3-CH_2-$ | | $C_2H_5-$ | 2 | |
| 36 | $CH_3-$ | $-CH_2-(CH_2)_3-CH_2-$ | | $i-C_3H_7-$ | 0 | |
| 37 | $CH_3-$ | $-CH_2-(CH_2)_3-CH_2-$ | | $i-C_3H_7-$ | 1 | |
| 38 | $CH_3-$ | $-CH_2-(CH_2)_3-CH_2-$ | | $i-C_3H_7-$ | 2 | |
| 39 | $CH_3-$ | $-CH_2-CH_2-CH_2-$ | | $i-C_3H_7-$ | 0 | $n_D^{20}$ = 1,5189 |
| 40 | $CH_3-$ | $-CH_2-CH_2-CH_2-$ | | $CH_3-$ | 0 | Smp.: 96-98°C |
| 41 | $CH_3-$ | $-CH_2-CH_2-CH_2-$ | | $i-C_3H_7-$ | 1 | Smp.: 97-99°C |
| 42 | $CH_3-$ | $-CH_2-CH_2-CH_2-$ | | $CH_3-$ | 0 | |
| 43 | $CH_3-$ | $-CH_2-(CH_2)_3-CH_2-$ | | $CH_3-$ | 0 | |
| 44 | $CH_3$ | $-CH_2-(CH_2)_3-CH_2-$ | | $CH_3-$ | 1 | |
| 45 | $CH_3-$ | $-CH_2-CH_2-CH_2-$ | | $CH_3-$ | 1 | Smp.:105-107°C |
| 46 | $n-C_4H_9-$ | $CH_3-$ | $CH_3-$ | $i-C_3H_7-$ | 0 | $n_D^{20}$ = 1,4919 |
| 47 | $s-C_4H_9-$ | $CH_3-$ | $CH_3-$ | $i-C_3H_7-$ | 0 | $n_D^{20}$ = 1,4943 |
| 48 | $i-C_4H_9-$ | $CH_3-$ | $CH_3-$ | $i-C_3H_7-$ | 0 | |
| 49 | $t-C_4H_9-$ | $CH_3-$ | $CH_3-$ | $i-C_3H_7-$ | 0 | Smp.: 79-81°C |
| 50 | $n-C_5H_{11}-$ | $CH_3-$ | $CH_3-$ | $i-C_3H_7-$ | 0 | $n_D^{20}$ = 1,4931 |
| 51 | $n-C_3H_7-$ | $CH_3-$ | $CH_3-$ | $i-C_3H_7-$ | 0 | $n_D^{20}$ = 1,4941 |
| 52 | $n-C_4H_9-$ | $CH_3-$ | $CH_3-$ | $i-C_3H_7-$ | 0 | $n_D^{20}$ = 1,5065 |

(Fortsetzung)

| Verbindung Nr. | $R_1$ | $R_2$ | $R_3$ | $R_4$ | n | physikalische Daten |
|---|---|---|---|---|---|---|
| 53 | $s-C_4H_9-$ | $CH_3-$ | $CH_3-$ | $i-C_3H_7-$ | 1 | $n_D^{20}$ = 1,5071 |
| 54 | $t-C_4H_9-$ | $CH_3-$ | $CH_3-$ | $i-C_3H_7-$ | 1 | Smp.: 80-82°C |
| 55 | $n-C_5H_{11}-$ | $CH_3-$ | $CH_3-$ | $i-C_3H_7-$ | 1 | $n_D^{20}$ = 1,5039 |
| 56 | $n-C_3H_7-$ | $CH_3$ | $CH_3-$ | $i-C_3H_7-$ | 1 | $n_D^{20}$ = 1,5068 |
| 57 | $s-C_4H_9-$ | $CH_3$ | $CH_3-$ | $CH_3-$ | 0 | $n_D^{20}$ = 1,5011 |
| 58 | $i-C_4H_9-$ | $CH_3-$ | $CH_3-$ | $CH_3-$ | 0 | $n_D^{20}$ = 1,5002 |
| 59 | $t-C_4H_9-$ | $CH_3-$ | $CH_3-$ | $CH_3-$ | 0 | Smp.: 78-82°C |
| 60 | $n-C_5H_{11}-$ | $CH_3-$ | $CH_3-$ | $CH_3-$ | 0 | $n_D^{20}$ = 1,4994 |
| 61 | $n-C_3H_7-$ | $CH_3-$ | $CH_3-$ | $CH_3-$ | | $n_D^{20}$ = 1,5049 |
| 62 | $s-C_4H_9-$ | $CH_3-$ | $CH_3-$ | $CH_3-$ | 1 | $n_D^{20}$ = 1,5152 |
| 63 | $i-C_4H_9-$ | $CH_3-$ | $CH_3-$ | $CH_3-$ | 1 | $n_D^{20}$ = 1,5102 |
| 64 | $t-C_4H_9-$ | $CH_3-$ | $CH_3-$ | $CH_3-$ | 1 | Smp.: 101-103°C |
| 65 | $n-C_5H_{11}-$ | $CH_3-$ | $CH_3-$ | $CH_3-$ | 1 | $n_D^{20}$ = 1,5059 |
| 66 | $n-C_3H_7-$ | $CH_3-$ | $CH_3-$ | $CH_3-$ | 1 | Smp.: 60-62°C |
| 67 | $C_2H_5-$ | $CH_3-$ | $CH_3-$ | $i-C_3H_7-$ | 1 | $n_D^{20}$ = 1,5100 |
| 68 | $i-C_3H_7-$ | $CH_3-$ | $CH_3-$ | $i-C_3H_7-$ | 1 | $n_D^{20}$ = 1,5068 |

Beispiel 4:

Formulierungen für flüssige Wirkstoffe der Formel I gemäss den Beispielen 1 bis 3 oder Kombinationen dieser Wirkstoffe mit andern Insektiziden oder Akariziden (% = Gewichtsprozent)

| 1. Emulsions-Konzentrate | a) | b) | c) |
|---|---|---|---|
| Wirkstoff resp. Wirkstoffkombination | 25 % | 40 % | 50 % |
| Ca Dodecylbenzolsulfonat | 5 % | 8 % | 6 % |
| Ricinusölpolyäthylenglykoläther (36 Mol AeO) | 5 % | - | - |
| Tributylphenolpolyäthylenglykoläther (30 Mol AeO) | - | 12 % | 4 % |
| Cyclohexan | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2. Lösungen | a) | b) | c) | d) |
|---|---|---|---|---|
| Wirkstoff resp. Wirkstoffkombination | 80 % | 10 % | 5 % | 95 % |
| Aethylenglykolmonomethyläther | 20 % | - | - | - |
| Polyäthylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| 3. Granulate | a) | b) |
|---|---|---|
| Wirkstoff resp. Wirkstoffkombination | 5 % | 10 % |
| Kaolin | 94 % | - |
| Hochdisperse Kieselsäure | 1 % | - |
| Attapulgit | - | 90 % |

Der Wirkstoff bzw. die Wirkstoffkombination wird in Methylenchlorid gelöst, auf den Träger aufgesprüht und das Lösungsmittel anschliessend im Vakuum abgedampft.

| 4. Stäubemittel | a) | b) |
|---|---|---|
| Wirkstoff resp. Wirkstoffkombination | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff bzw. der Wirkstoffkombination erhält man gebrauchsfertige Stäubemittel.

Formulierungen für feste Wirkstoffe der Formel I gemäss den Beispielen 1 bis 3 resp. Kombinationen dieser Wirkstoffe mit andern Insektiziden oder Akariziden (% = Gewichtsprozent)

| 1. Spritzpulver | a) | b) | c) |
|---|---|---|---|
| Wirkstoff oder Wirkstoffkombination | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 2. Emulsions-Konzentrat | |
|---|---|
| Wirkstoff oder Wirkstoffkombination | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

3. <u>Stäubemittel</u>

| | a) | b) |
|---|---|---|
| Wirkstoff oder Wirkstoffkombination | 5 % | 8 % |
| Talkum | 95 % | - |
| Kaolin | - | 92 % |

Man erhält anwendungsfertige Stäubemittel, indem der Wirkstoff oder die Wirkstoffkombination mit dem Träger vermischt und auf einer geeigneten Mühle vermahlen wird.

4. <u>Extruder-Granulat</u>

| | |
|---|---|
| Wirkstoff oder Wirkstoffkombination | 10 % |
| Na-Ligninsulfonat | 2 % |
| Carboxymethylcellulose | 1 % |
| Kaolin | 87 % |

Der Wirkstoff oder die Wirkstoffkombination wird mit den Zusatzstoffen vermischt, vermahlen und mit Wasser angefeuchtet. Dieses Gemisch wird extrudiert, granuliert und anschliessend im Luftstrom getrocknet.

5. <u>Umhüllungs-Granulat</u>

| | |
|---|---|
| Wirkstoff oder Wirkstoffkombination | 3 % |
| Polyäthylenglykol (MG 200) | 3 % |
| Kaolin | 94 % |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

6. Suspensions-Konzentrat

| | |
|---|---|
| Wirkstoff oder Wirkstoffkombination | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37 %ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 5: Wirkung gegen Musca domestica

Je 50 g frisch zubereitetes CSMA-Nährsubstrat für Maden werden in Becher eingewogen. Von einer 1 Gew.-%igen acetonischen Lösung des betreffenden Wirkstoffes wird eine bestimmte Menge auf das in den Bechern befindliche Nährsubstrat pipettiert, sodass sich eine Wirkstoffkonzentration von 800 ppm ergibt. Nach dem Durchmischen des Substrates lässt man das Aceton mindestens 20 Stunden lang verdampfen.

Dann werden pro Wirkstoff und Konzentration je 25 eintägige Maden von Musca domestica in die das so behandelte Nährsubstrat enthaltenden Becher gegeben. Nachdem sich die Maden verpuppt haben, werden die gebildeten Puppen durch Ausschwemmen mit Wasser von dem Substrat abgetrennt und in mit Siebdeckeln verschlossenen Gefässen deponiert.

Die pro Ansatz ausgeschwemmten Puppen werden gezählt (toxischer Einfluss des Wirkstoffes auf die Madenentwicklung). Dann wird nach 10 Tagen die Anzahl der aus den Puppen geschlüpften Fliegen bestimmt.

Verbindungen der Formel I gemäss den Beispielen 1 bis 3 zeigen gute Wirkung im obigen Test.

Beispiel 6: Wirkung gegen Lucilia sericata

Zu 9 ml eines Zuchtmediums wird bei 50°C 1 ml einer 0,5 % Aktivsubstanz enthaltenden wässrigen Zubereitung gegeben. Nun werden ca. 30 frisch geschlüpfte Lucilia sericata-Larven zum Zuchtmedium hinzugefügt. Nach 48 und 96 Stunden wird die insektizide Wirkung durch Ermittlung der Abtötungsrate festgestellt.

Verbindungen der Formel I gemäss den Beispielen 1 bis 3 zeigen in diesem Test gute Wirkung gegen Lucilia sericata.

Beispiel 7: Wirkung gegen Aëdes aegypti

Auf die Oberfläche von 150 ml Wasser, das sich in einem Behälter befindet, wird so viel einer 0,1%igen acetonischen Lösung des Wirkstoffes pipettiert, dass eine Konzentration von 800 ppm erhalten wird. Nach Verdunsten des Acetons wird der Behälter mit 30 bis 40 2-tägigen Aëdes-Larven beschickt. Nach 2 und 7 Tagen wird die Mortalität geprüft.

Verbindungen der Formel I gemäss den Beispielen 1 bis 3 zeigen gute Wirkung im obigen Test.

Beispiel 8: Reproduktions-Beeinflussung von Anthonomus grandis

Adulte Anthonomus grandis, die nach dem Schlupf nicht älter als 24 Stunden waren, werden in Gruppen zu jeweils 25 Käfern in Käfige mit Gitterwänden überführt. Die mit den Käfern besetzten Käfige werden sodann während 5 bis 10 Sekunden in eine acetonische Lösung, enthaltend 800 ppm des zu prüfenden Wirkstoffes eingetaucht. Nachdem die Käfer trocken sind, werden sie zur Kopulation und Eiablage in abgedeckte und Futter enthaltende Schalen eingesetzt. Abgelegte Eier werden zwei- bis dreimal wöchentlich mit fliessendem Wasser ausgeschwemmt, gezählt, durch zwei- bis dreistündiges Einlegen in ein

wässriges Desinfektionsmittel desinfiziert und dann in Schalen, die eine geeignete Larvaldiät enthalten, deponiert. Nach 7 Tagen wird die %-Mortalität der Eier bestimmt, d.h. wieviel Larven sich aus den deponierten Eiern entwickelt haben.

Zur Ermittlung der Dauer des die Reproduktion beeinflussenden Effektes der zu prüfenden Wirkstoffe wird die Eiablage der Käfer noch weiter, d.h. während eines Zeitraums von etwa 4 Wochen überprüft. Die Bonitierung erfolgt anhand der Verminderung der Anzahl abgelegter Eier und daraus geschlüpfter Larven im Vergleich zu unbehandelten Kontrollen.

Verbindungen der Formel I gemäss den Beispielen 1 bis 3 zeigen gute Wirkung in obigem Test.

Beispiel 9: Kontaktwirkung gegen Aphis craccivora

In Töpfen angezogene 4-5 Tage alte Erbsenkeimlinge (Vicia faba) werden vor Versuchsbeginn mit je ca. 200 Individuen der Spezies Aphis craccivora besiedelt. Die so behandelten Pflanzen werden 24 Stunden später mit einer wässrigen Zubereitung enthaltend 12,5 ppm der zu prüfenden Verbindung bis zur Tropfnässe direkt besprüht. Man verwendet pro Test-Verbindung zwei Pflanzen. Eine Auswertung der erzielten Abtötungsrate erfolgt nach weiteren 24 und 72 Stunden. Der Versuch wird bei 21-22°C und einer rel. Luftfeuchtigkeit von etwa 55 % durchgeführt.

Die Verbindungen Nr. 1 und 2 gemäss den Beispielen 1 und 2 ergeben eine Mortalität von 90-100 % in diesem Test.

Beispiel 10: Systemische Wirkung gegen Aphis craccivora (in Wasser)

Etwa 1-2 cm grosse Erbsenkeimlinge, die 24 Stunden vor Beginn des Versuches mit einer Population der Blattläuse infestiert worden waren, werden in 20 ml einer wässrigen Brühe gestellt, die den zu prüfenden Wirkstoffes enthält. Die wässrige Brühe wird aus einem Emulsionskonzentrat oder einer benetzbaren Pulverzubereitung des betreffenden Wirkstoffes hergestellt und befindet sich in einem

Gefäss, dass mit einem Löcher aufweisenden Plastikdeckel abgeschlossen ist. Die Wurzel der infestierten Erbsenpflanze wird jeweils durch ein Loch des Plastikdeckels in die Brühe geschoben. Das Loch wird dann mit Watte abgedichtet, um die Pflanze zu fixieren und einen eventuellen Einfluss der Gasphase aus der Brühe auszuschalten.

Der Versuch wird bei 20°C und 60 % relativer Luftfeuchtigkeit durchgeführt. Nach zwei Tagen wird auf die Anzahl der nicht mehr saugfähigen Testtiere, im Vergleich zu unbehandelten Kontrollen bonitiert. Damit wird festgestellt, ob der über die Wurzel aufgenommene Wirkstoff die Blattläuse an den oberen Pflanzenteilen abtötet.

Die Verbindungen Nr. 1 und 2 gemäss den Beispielen 1 bzw. 2 zeigen in obigem Versuch bei 12,5 bzw. 0,75 ppm 90-100%-ige Wirkung (Mortalität) gegen Aphis craccivora.

Beispiel 11: Kontaktwirkung gegen Myzus persicae

Etwa 4 bis 5 Tage alte, in Wasser angezogene Erbsenkeimlinge (Vicia faba) werden vor Versuchsbeginn jeweils mit ca. 200 Individuen der Spezies Myzus persicae besiedelt. Die so behandelten Pflanzen werden 24 Stunden später mit einer wässrigen Suspension enthaltend 50 bzw.100 ppm der zu prüfenden Verbindung bis zur Tropfnässe direkt besprüht. Man verwendet pro Testsubstanz zwei Pflanzen. Eine Auswertung der erzielten Abtötungsrate erfolgt 24 und 72 Stunden nach Applikation. Der Versuch wird bei 21-22°C und etwa 60 % relativer Luftfeuchtigkeit durchgeführt.

Die Verbindungen Nr. 1 und 2 gemäss den Beispielen 1 bzw. 2 zeigen 90-100 %ige Mortalität in diesem Test.

Beispiel 12: Systemische Wirkung gegen Myzus persicae (in Erde)

Bewurzelte Kohlpflanzen im 4- bis 5-Blatt-Stadium werden in Töpfe, welche 60 ccm Erde enthalten, verpflanzt. Ansschliessend werden 50 ml einer wässrigen Formulierung der zu prüfenden Verbindung

(erhalten aus einem 25%igen Spritzpulver) jeweils in einer Konzentration von 800 ppm direkt auf die Erde aufgegossen, ohne die Pflanze selbst zu benetzen.

Nach 24 Stunden werden auf die oberirdischen Pflanzenteile der behandelten Pflanzen Blattläuse der Spezies Myzus persicae gesetzt und die Pflanzen mit Plastikzylindern überdeckt, um die Blattläuse vor einer eventuellen direkten Kontakt- oder Gaswirkung der Testsubstanz zu schützen.

Die Auswertung der erzielten %-Abtötung erfolgt 48 Stunden nach Versuchsbeginn. Pro Testsubstanz werden zwei Pflanzen, je eine in separaten Töpfen, verwendet. Der Versuch wird bei ca. 25°C und 60 % relativer Luftfeuchtigkeit durchgeführt.

Die Verbindungen der Formel I gemäss den Beispielen 1 bis 3 zeigen gute Wirkung in obigem Test.

Beispiel 13: Blattpenetrations-Wirkung gegen Aphis craccivora

In etwa 8 cm hohe Kunststoffbecher (Durchmesser etwa 6 cm) wird ein passender kleiner Zweig von Vicia faba gelegt, der mit Blattläusen der Spezies Aphis craccivora stark infiziert ist. Der Becher wird mit einem Kunststoffdeckel, der in der Mitte eine ausgestanzte Oeffnung von 2 cm Durchmesser aufweist, bedeckt. Auf die in dem Deckel befindliche Oeffnung wird ein Blatt einer Vicia faba-Pflanze gelegt, ohne dieses Blatt von der eingetopften Pflanze abzutrennen. Das Blatt wird dann mit einem zweiten Lochdeckel auf dem Becher über der Oeffnung des ersten Deckels fixiert. Von der Unterseite her, d.h. durch die Oeffnung des ersten Deckels hindurch, infizieren nun die in dem Becher befindlichen Blattläuse das obenliegende Blatt der Futterpflanze. Auf der Oberseite wird auf das Blatt eine wässrige Zubereitung des zu prüfenden Wirkstoffes in einer Konzentration von 800 ppm mittels eines Pinsels gleichmässig aufgetragen. Es wird geprüft, ob die einseitig auf die Oberseite des Blattes der Futterpflanze aufgetragene Testsubstanz in genügender Menge durch das Blatt hindurch auf dessen Unterseite diffundiert, um dort saugende Blattläuse abzutöten.

Der Versuch wird bei etwa 20°C und 60 % relativer Luftfeuchtigkeit durchgeführt. Die Auswertung auf %-Mortalität erfolgt 48 Stunden nach Wirkstoffapplikation.

Verbindungen der Formel I gemäss den Beispielen 1 bis 3 zeigen gute Wirkung in obigem Test.

Beispiel 14: Wirkung gegen Diabrotica balteata (in Erde)
Es werden 350 ml Erde (bestehend aus 95 Vol.-% Sand und 5 Vol.-% Torf) mit 150 ml einer wässrigen Emulsionszubereitung vermischt, die den zu prüfenden Wirkstoff in einer Konzentration von 12,5 ppm enthält. Dann werden Plastikbecher, die einen oberen Durchmesser von ca. 10 cm haben mit der so behandelten Erde teilweise gefüllt. Pro Becher werden zehn Larven von Diabrotica balteata im dritten Larvalstadium eingesetzt, vier Maiskeimlinge eingepflanzt und die Becher mit Erde aufgefüllt. Die gefüllten Becher werden mit Plastikfolie abgedeckt und bei einer Temperatur von etwa 22°C gehalten. Zehn Tage nach dem Ansatz wird die in den Bechern enthaltene Erde abgesiebt und die zurückbleibenden Larven hinsichtlich ihrer Mortalität geprüft.

Die Verbindung Nr. 1 gemäss Beispiel 1 zeigt in diesem Test eine Wirkung von 80-100 % (Mortalität).

Beispiel 15: Wirkung gegen Diabrotica balteata (in Wasser)
Es werden 5 Maiskeimlinge von 1 -3 cm sowie eine Filterpapier-Rondelle in eine wässrige Lösung enthaltend den Wirkstoff in einer Konzentration von 12,5 ppm und etwa 4 Vol.-% Aceton eingetaucht. Die eingetauchte Filterpapier-Rondelle wird auf den Boden eines Kunststoff-Bechers (Inhalt 200 ml) gelegt und darauf wird eine trockene Filterpapier-Rondelle sowie die Maiskeimlinge und 10 Larven von Diabrotica balteata im 2. oder 3. Larvalstadium gegeben. Der

Ansatz wird bei ca. 24°C und 40 - 60 % relativer Luftfeuchtigkeit und Tageslicht gehalten. Die Bonitur erfolgt nach 6 Tagen gegenüber unbehandelten Kontrollansätzen.

Verbindung Nr. 1 gemäss Beispiel 1 zeigt in diesem Test eine Wirkung von 80-100 % (Mortalität).

Beispiel 16: Wirkung gegen Laodelphax striatellus und Nilaparvata lugens (Nymphen)

Der Test wird an wachsenden Pflanzen durchgeführt. Dazu werden jeweils 4 Reispflanzen (Dicke des Stengels 8 mm) mit einer Höhe von ca. 20 cm in Töpfe (Durchmesser von 8 cm) eingepflanzt.

Die Pflanzen werden auf einem Drehteller mit jeweils 100 ml einer acetonischen Lösung enthaltend 400 ppm des zu prüfenden Wirkstoffs besprüht. Nach dem Antrocknen des Spritzbelages erfolgt die Besiedlung jeder Pflanze mit je 20 Nymphen der Testtiere im dritten Stadium. Um die Zikaden am Entweichen zu hindern, wird über die besiedelten Pflanzen jeweils ein Glaszylinder gestülpft und dieser mit einem Gaze-Deckel abgedeckt. Die Nymphen werden bis zum Erreichen des folgenden Entwicklungsstadiums über 10 Tage an der behandelten Pflanze gehalten. Die Auswertung auf %-Mortalität erfolgt 1, 4 und 8 Tage nach der Behandlung.

Die erfindungsgemässen Verbindungen Nr. 1 und 2 gemäss den Beispielen 1 bzw. 2 zeigen in diesem Test 80-100 % Wirksamkeit (Mortalität) gegen Nilaparvata bei 400 ppm bzw. 200 ppm.

Die erfindungsgemässen Verbindungen der Formel I zeigen in diesem Test auch gute Wirksamkeit gegen Laodelphax striatellus.

Beispiel 17: Systemische Wirkung gegen Nilaparvata lugens (Nymphen)

Der Test wird an wachsenden, 10 bis 14 Tagen alten Reispflanzen durchgeführt, die sich in mit Erde gefüllten Pflanzbechern von 5,5 cm Durchmesser befinden.

Die in den Pflanzbechern befindliche Erde wird mit 5 ml einer wässrigen Emulsionszubereitung angegossen, die die jeweils zu prüfende Verbindung in einer Konzentration von 12,5 ppm enthält. Nach einer Woche werden die so behandelten Pflanzen mit jeweils 20 Nymphen von Nilaparvata lugens im N3-Stadium besiedelt. Sechs Tage nach der Besiedlung wird die %-Mortalität der Test-Insekten gegenüber unbehandelten Kontrollansätzen bestimmt.

Der Versuch wird bei etwa 23°C, 60 % relativer Luftfeuchtigkeit und 14-stündigem Belichtungszeitraum pro Tag durchgeführt.

Die Verbindungen Nr. 1 und 2 gemäss den Beispielen 1 bzw. 2 zeigen bei 12,5 ppm 80-100 %-ige Wirkung (Mortalität) in diesem Test.

<u>Beispiel 18:</u> <u>Potenzierung der insektiziden Wirkung gegen Myzus persicae</u>

Die folgenden Testverbindungen wurden verwendet:

<u>Verbindung A:</u> erfindungsgemässe Verbindung Nr. 1 gemäss Beispiel 1 der Formel

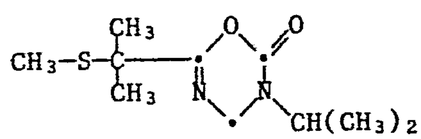

als 20%iges Spritzpulver;

<u>Verbindung B:</u> Phosphamidon der Formel

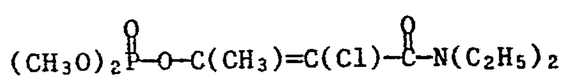

(beschrieben in US-PS Nr. 2.908.605) als 20%iges Spritzpulver.

<u>Testdurchführung:</u>

Etwa 4 cm hohe, in Wasser angezogene Peperoni- bzw. Erbsenkeimlinge werden vor dem Versuchsbeginn jeweils mit ca. 200 Individuen der Spezies Myzus persicae besiedelt. Die so behandelten Pflanzen werden

24 Stunden später mit wässrigen Suspensionen enthaltend ansteigende Mengen der zu prüfenden Verbindungen A und B oder deren Kombination bis zur Tropfnässe besprüht. Man verwendet pro Konzentration zwei Pflanzen. Eine Auswertung der erzielten Abtötungsrate (% Mortalität) erfolgt 48 Stunden nach Wirkstoff-Applikation. Der Versuch wird im Gewächshaus bei 20-22°C und 60 % relativer Luftfeuchtigkeit durchgeführt.

Testergebnis:

| verwendete Verbindungen | %-Mortalität (auf Peperoni) Wirkstoffkonzentration | | |
|---|---|---|---|
| | 100 ppm | 50 ppm | 25 ppm |
| Verbindung A | 0 % | 0 % | 0 % |
| Verbindung B | 58 % | 0 % | 0 % |
| Kombination A + B* | 100 % | 94 % | 82 % |

* (1:1; d.h. je 50/25/12,5 ppm von beiden Komponenten A und B in der Spritzlösung)

| verwendete Verbindungen | %-Mortalität (auf Erbsen) Wirkstoffkonzentration | | |
|---|---|---|---|
| | 400 ppm | 200 ppm | 100 ppm |
| Verbindung A | 65 % | 0 % | 0 % |
| Verbindung B | 88 % | 0 % | 0 % |
| Kombination A + B** | 100 % | 100 % | 88 % |

** (1:1; d.h. je 200/100/50 ppm von beiden Komponenten A und B in der Spritzlösung)

Beispiel 19: Wirkung gegen Nephotettix cincticeps (Nymphen)

Der Test wird an wachsenden Pflanzen durchgeführt. Dazu werden ca. 20 Tage alte Reispflanzen mit einer Höhe von etwa 15 cm in Töpfe (Durchmesser 5,5 cm) eingepflanzt.

Die Pflanzen werden auf Drehtellern mit jeweils 100 ml einer acetonischen Lösung enthaltend 12,5, 50, 100, 200 oder 400 ppm des Wirkstoffs Nr. 2 besprüht. Nach dem Antrocknen des Spritzbelages erfolgt die Besiedlung jeder Pflanze mit je 20 Nymphen der Testtiere im zweiten oder dritten Stadium. Um die Zikaden am Entweichen zu hindern, wird über die besiedelten Pflanzen jeweils ein Plexiglaszylinder gestülpt und dieser mit einem Gaze-Deckel abgedeckt. Die Nymphen werden für 5 Tage an der behandelten Pflanze, die mindestens 1 mal nachgegossen werden muss, gehalten. Der Versuch wird bei einer Temperatur von ca. 23°C, bei 55 % relativer Luftfeuchtigkeit und mit einer Belichtungsperiode von 16 Stunden durchgeführt.

Am sechsten Tag wird an den mit 50 ppm oder mehr Wirkstoff besprühten Pflanzen eine mindestens 80%-ige Mortalität der Testtiere festgestellt.

Patentansprüche (für alle benannten Vertragsstaaten ausser AT und ES)

1. Verbindung der Formel I

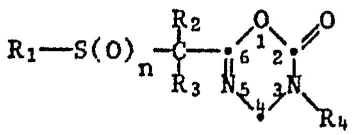 (I),

worin

$R_1$ und $R_4$ unabhängig voneinander $C_1$-$C_8$-Alkyl, $C_3$-$C_6$-Cycloalkyl, $C_3$-$C_4$-Alkenyl, $C_3$-$C_4$-Alkinyl, Phenyl oder Benzyl;

$R_2$ und $R_3$ unabhängig voneinander Wasserstoff, $C_1$-$C_4$-Alkyl oder $R_2$ und $R_3$ zusammen mit dem Kohlenstoffatom, an das sie gebunden sind, $C_3$-$C_6$-Cycloalkyl bedeuten; und

n = 0, 1 oder 2 ist.

2. Verbindung der Formel I gemäss Anspruch 1, dadurch gekennzeichnet, dass

$R_1$ $C_1$-$C_4$-Alkyl oder Phenyl;

$R_2$ und $R_3$ Methyl;

$R_4$ $C_1$-$C_4$-Alkyl bedeuten; und

n = 0, 1 oder 2 ist.

3. Verbindung der Formel I gemäss Anspruch 2, dadurch gekennzeichnet, dass

$R_1$ Methyl oder Aethyl;

$R_2$ und $R_3$ Methyl:

$R_4$ $C_1$-$C_3$-Alkyl bedeuten; und

n = 0 oder 1 ist.

4. Verbindung gemäss Anspruch 3 der Formel

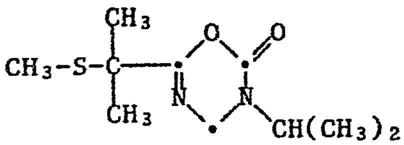

.

5. Verbindung gemäss Anspruch 3 der Formel

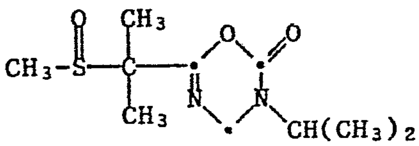

.

6. Verbindung gemäss Anspruch 2 der Formel

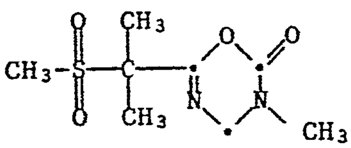

.

7. Verbindung gemäss Anspruch 3 der Formel

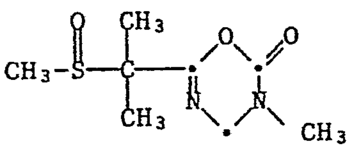

.

8. Verbindung gemäss Anspruch 3 der Formel

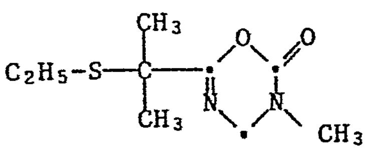

.

9. Verbindung gemäss Anspruch 3 der Formel

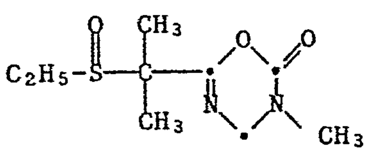

.

10. Verfahren zur Herstellung einer Verbindung der Formel I gemäss einem der Ansprüche 1 bis 9, dadurch gekennzeichnet, dass man eine Verbindung der Formel II

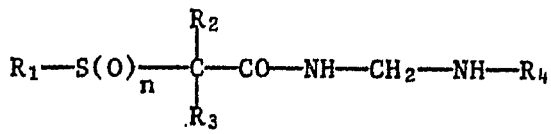

(II)

mit Phosgen umsetzt, wobei $R_1$ bis $R_4$ und n die in einem der Ansprüche 1 bis 3 angegebenen Bedeutungen haben.

11. Verfahren zur Herstellung einer Verbindung der Formel I gemäss einem der Ansprüche 1 bis 3, 5 bis 7 und 9 worin n = 1 oder 2 ist, dadurch gekennzeichnet, dass man eine Verbindung der Formel I, worin n = 0 ist, oxydiert.

12. Verfahren zur Herstellung einer Verbindung der Formel I gemäss einem der Ansprüche 1 bis 3 und 6, worin n = 2 ist, dadurch gekennzeichnet, dass man eine Verbindung der Formel I, worin n = 1 ist, oxydiert.

13. Schädlingsbekämpfungsmittel, welches als aktive Komponente eine Verbindung der Formel I gemäss den Ansprüchen 1 bis 9 zusammen mit geeigneten Trägern und/oder anderen Zuschlagstoffen enthält.

14. Mittel gemäss Anspruch 13 mit potenzierender bzw. synergistischer Wirkung enthaltend als aktive Komponente neben einer Verbindung der Formel I einen weiteren insektiziden und/oder akariziden Wirkstoff.

15. Mittel gemäss Anspruch 14 enthaltend als aktive Komponenten eine Verbindung der Formel I und Phosphamidon.

16. Verwendung einer Verbindung gemäss den Ansprüchen 1 bis 9 oder eines Mittels gemäss einem der Ansprüche 13 bis 15 zur Bekämpfung von Insekten und Vertretern der Ordnung Akarina an Tieren und Pflanzen.

17. Verwendung gemäss Anspruch 16 zur Bekämpfung pflanzenschädigender Insekten.

18. Verwendung gemäss Anspruch 17 zur Bekämpfung pflanzenschädigender saugender Insekten.

19. Verwendung gemäss Anspruch 17 in Reiskulturen.

20. Verfahren zur Bekämpfung von Insekten und Vertretern der Ordnung Akarina, dadurch gekennzeichnet, dass man die Schädlinge bzw. deren Eier und/oder verschiedene Entwicklungsstadien oder ihren Aufenthaltsort mit einer pestizid wirksamen Menge einer Verbindung der Formel I gemäss einem der Ansprüchen 1 bis 9 oder mit einem Mittel gemäss einem der Ansprüche 13 bis 15 in Kontakt bringt oder behandelt.

FO 7.5/IS/kg*

Patentansprüche (für Oesterreich und Spanien)

1. Schädlingsbekämpfungsmittel, welches als aktive Komponente eine Verbindung der Formel I

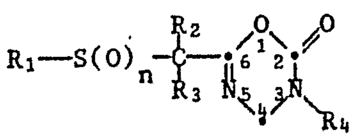

(I),

worin

$R_1$ und $R_4$ unabhängig voneinander $C_1$-$C_8$-Alkyl, $C_3$-$C_6$-Cycloalkyl, $C_3$-$C_4$-Alkenyl, $C_3$-$C_4$-Alkinyl, Phenyl oder Benzyl;

$R_2$ und $R_3$ unabhängig voneinander Wasserstoff, $C_1$-$C_4$-Alkyl oder $R_2$ und $R_3$ zusammen mit dem Kohlenstoffatom, an das sie gebunden sind, $C_3$-$C_6$-Cycloalkyl bedeuten; und

n = 0, 1 oder 2 ist, zusammen mit anderen Trägern und/oder Zuschlagsstoffen enthält.

2. Schädlingsbekämpfungsmittel gemäss Anspruch 1, dadurch gekennzeichnet, dass

$R_1$ $C_1$-$C_4$-Alkyl oder Phenyl;

$R_2$ und $R_3$ Methyl;

$R_4$ $C_1$-$C_4$-Alkyl bedeuten; und

n = 0, 1 oder 2 ist.

3. Schädlingsbekämpfungsmittel gemäss Anspruch 2, dadurch gekennzeichnet, dass

$R_1$ Methyl oder Aethyl;

$R_2$ und $R_3$ Methyl:

$R_4$ $C_1$-$C_3$-Alkyl bedeuten; und

n = 0 oder 1 ist.

4. Schädlingsbekämpfungsmittel gemäss Anspruch 3, dadurch gekennzeichnet, dass es als aktive Komponente die Verbindung der Formel

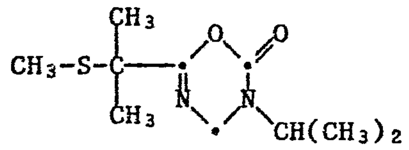

enthält.

5. Schädlingsbekämpfungsmittel gemäss Anspruch 3, dadurch gekennzeichnet, dass es als aktive Komponente die Verbindung der Formel

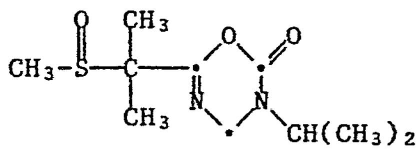

enthält.

6. Schädlingsbekämpfungsmittel gemäss Anspruch 3, dadurch gekennzeichnet, dass es als aktive Komponente die Verbindung der Formel

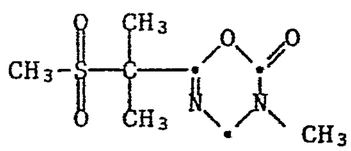

enthält.

7. Schädlingsbekämpfungsmittel gemäss Anspruch 3, dadurch gekennzeichnet, dass es als aktive Komponente die Verbindung der Formel

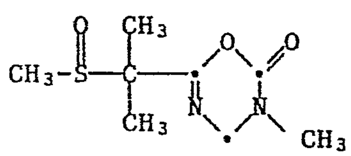

enthält.

8. Schädlingsbekämpfungsmittel gemäss Anspruch 3, dadurch gekennzeichnet, dass es als aktive Komponente die Verbindung der Formel

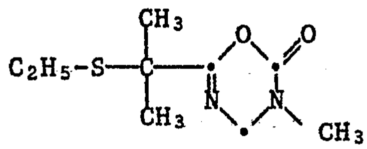

enthält.

9. Schädlingsbekämpfungsmittel gemäss Anspruch 3, dadurch gekennzeichnet, dass es als aktive Komponente die Verbindung der Formel

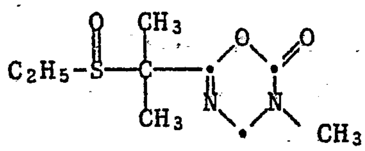

enthält.

10. Verfahren zur Herstellung einer Verbindung der Formel I gemäss einem der Ansprüche 1 bis 9, dadurch gekennzeichnet, dass man eine Verbindung der Formel II

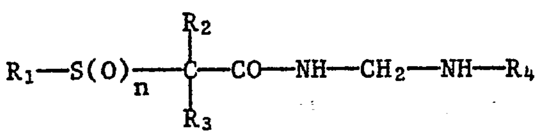

(II)

mit Phosgen umsetzt, wobei $R_1$ bis $R_4$ und n die in einem der Ansprüche 1 bis 3 angegebenen Bedeutungen haben.

11. Verfahren zur Herstellung einer Verbindung der Formel I gemäss einem der Ansprüche 1 bis 3, 5 bis 7 und 9 worin n = 1 oder 2 ist, dadurch gekennzeichnet, dass man eine Verbindung der Formel I, worin n = 0 ist, oxydiert.

12. Verfahren zur Herstellung einer Verbindung der Formel I gemäss einem der Ansprüche 1 bis 3 und 6, worin n = 2 ist, dadurch gekennzeichnet, dass man eine Verbindung der Formel I, worin n = 1 ist, oxydiert.

13. Mittel gemäss Anspruch 1 mit potenzierender bzw. synergistischer Wirkung enthaltend als aktive Komponente neben einer Verbindung der Formel I einen weiteren insektiziden und/oder akariziden Wirkstoff.

14. Mittel gemäss Anspruch 13 enthaltend als aktive Komponenten eine Verbindung der Formel I und Phosphamidon.

15. Verwendung einer Verbindung gemäss den Ansprüchen 1 bis 9 oder eines Mittels gemäss einem der Ansprüche 13 und 14 zur Bekämpfung von Insekten und Vertretern der Ordnung Akarina an Tieren und Pflanzen.

16. Verwendung gemäss Anspruch 15 zur Bekämpfung pflanzenschädigender Insekten.

17. Verwendung gemäss Anspruch 16 zur Bekämpfung pflanzenschädigender saugender Insekten.

18. Verwendung gemäss Anspruch 16 in Reiskulturen.

19. Verfahren zur Bekämpfung von Insekten und Vertretern der Ordnung Akarina, dadurch gekennzeichnet, dass man die Schädlinge bzw. deren Eier und/oder verschiedene Entwicklungsstadien oder ihren Aufenthaltsort mit einer pestizid wirksamen Menge einer Verbindung der Formel I gemäss einem der Ansprüchen 1 bis 9 oder mit einem Mittel gemäss einem der Ansprüche 13 und 14 in Kontakt bringt oder behandelt.

FO 7.5/IS/kg*